# EUROPEAN PATENT SPECIFICATION

(11) **EP 2 614 144 B1**
(45) Date of publication and mention of the grant of the patent: **22.07.2015**
(21) Application number: 11770339.7
(22) Date of filing: 08.09.2011
(51) Int. Cl.: C12N 9/20, A61K 31/00

(54) **USE OF INHIBITORS OF PHOSPHOLIPASE A2 FOR THE TREATMENT OR PREVENTION OF FLAVIVIRUS INFECTION**
VERWENDUNG VON PHOSPHOLIPASE-A2-HEMMERN ZUR BEHANDLUNG ODER PRÄVENTION EINER FLAVIVIRUS-INFEKTION
UTILISATION D'INHIBITEURS DE PHOSPHOLIPASE A2 POUR TRAITER OU PRÉVENIR UNE INFECTION À FLAVIVIRUS

(30) Priority: 08.09.2010 US 380788 P
(43) Date of publication of application: 17.07.2013
(73) Proprietor: Twincore Zentrum für Experimentelle und Klinische Infektionsforschung GmbH, 30625 Hannover (DE); Ruprecht-Karls-Universität Heidelberg, 69117 Heidelberg (DE)
(72) Inventor: MENZEL, Nicolas, 40670 Meerbusch (DE); PIETSCHMANN, Thomas, 30559 Hannover (DE); BARTENSCHLAGER, Ralf, 69120 Heidelberg (DE); FISCHL, Wolfgang, 69120 Heidelberg (DE)
(74) Representative: Forstmeyer, Dietmar
(86) International application number: PCT/EP2011/004532
(87) International publication number: WO 2012/031763

(56) References cited:
- CHOCKALINGAM KARUPPIAH ET AL: "A cell protection screen reveals potent inhibitors of multiple stages of the hepatitis C virus life cycle.", PROCEEDINGS OF THE NATIONAL ACADEMY OF SCIENCES OF THE UNITED STATES OF AMERICA 23 FEB 2010 LNKD- PUBMED:20142494, vol. 107, no. 8, 23 February 2010 (2010-02-23), pages 3764-3769, XP002663299, ISSN: 1091-6490
- GASTAMINZA PABLO ET AL: "Unbiased probing of the entire hepatitis C virus life cycle identifies clinical compounds that target multiple aspects of the infection.", PROCEEDINGS OF THE NATIONAL ACADEMY OF SCIENCES OF THE UNITED STATES OF AMERICA 5 JAN 2010 LNKD- PUBMED:19995961, vol. 107, no. 1, 5 January 2010 (2010-01-05), pages 291-296, XP002663300, ISSN: 1091-6490
- YAMAMOTO MINA ET AL: "Inhibitory effect of a potent and selective cytosolic phospholipase A2alpha inhibitor RSC-3388 on skin inflammation in mice", PHARMACOLOGY, S. KARGER AG, CH, vol. 81, no. 4, 1 January 2008 (2008-01-01), pages 301-311, XP009153914, ISSN: 1423-0313 cited in the application
- DENNIS E A: "DIVERSITY OF GROUP TYPES, REGULATION, AND FUNCTION OF PHOSPHOLIPASEA2", JOURNAL OF BIOLOGICAL CHEMISTRY, THE AMERICAN SOCIETY OF BIOLOGICAL CHEMISTS, INC, US, vol. 269, no. 18, 6 May 1994 (1994-05-06), pages 13057-13060, XP002923605, ISSN: 0021-9258 cited in the application
- MAGRIOTI VICTORIA ET AL: "Phospholipase A2 inhibitors as potential therapeutic agents for the treatment of inflammatory diseases", EXPERT OPINION ON THERAPEUTIC PATENTS, INFORMA HEALTHCARE, UNITED KINGDOM, vol. 20, no. 1, 1 January 2010 (2010-01-01), pages 1-18, XP009153943, ISSN: 1744-7674 cited in the application
- CLARK J D ET AL: "Potential therapeutic uses of phospholipase A2 inhibitors", EXPERT OPINION ON THERAPEUTIC PATENTS, INFORMA HEALTHCARE, GB, vol. 14, no. 7, 1 January 2004 (2004-01-01), pages 937-950, XP002405708, ISSN: 1354-3776, DOI: 10.1517/13543776.14.7.937 cited in the application

## Description

### Field of the Invention

The invention relates to an inhibitor of cytosolic phospholipase A2 alpha (hereinafter also referred to as cPLA2α or cPLA2a inhibitor) for use in the treatment or prevention of flavivirus infection, in particular an infection with a flavivirus of the genus Flavi or the genus Hepaci. The invention also provides a pharmaceutical formulation and a combination preparation for use in the treatment or prevention of an infection with a flavivirus of the genus Flavi or the genus Hepaci.

### Background of the Invention

### Phospholipase A2

Phospholipases A2 (PLA2s) are a superfamily of key enzymes involved in a multitude of (patho)physiological and cellular processes (Balsinde et al., (1999) Annu. Rev. Pharmacol. Toxicol. 39: 175-189 ; Kramer and Sharp (1997) FEBSLetters 410: 49-53; Nishizuka (1992) Science 258: 607-614 ; Dennis (1994) R Biol. Chem. 269: 13057-13060 ; Dennis (1997) Trends Biochem. Sci. 22: 1-2; Yuan and Tsai (1999) Biochim Biophys Acta 1441: 215). Phospholipases A2 (PLA2s) constitute one of the largest families of lipolytic enzymes and are defined by their ability to catalyze the hydrolysis of the ester bond at the sn-2 position of glycerophospholipids, yielding free fatty acids and lysophospholipids, from which secondary messengers may be generated. In vivo, the sn-2 position of phospholipids frequently contains polyunsaturated fatty acids that may be metabolized to form various eicosanoids and related bioactive lipids. Lysophospholipids also have various important roles in biological processes. The PLA2 superfamily currently consists of fifteen groups and many subgroups of which a number of enzymes differ in primary sequence, structure and catalytic mechanism. There are four main types or classes of PLA2: the secreted sPLA2, the cytosolic cPLA2, the Ca2+-independent iPLA2 and the lipoprotein associated LpPLA2. A general overview of the PLA2 superfamily can, for example, be found in review articles of Burke and Dennis (J Lipid Res 2009; 50: 237-42; Cardiovasc Drugs Ther 2009; 23: 45-59) and Schaloske and Dennis (Biochim Biophys Acta 2006; 1761: 1246-59).

The cytosolic PLA2s are large proteins with variable sizes (61 - 114 kDa) and do not possess the same disulfide network as the sPLA2s. Groups IVA, IVB, IVC, IVD, IVE and IVF are included in this category. The first Group IVA cytosolic enzyme was identified in human platelets in 1986 and was cloned and sequenced in 1991 (Rouault et al., Biochemistry 2007; 46: 1647-62; Kramer et al., Biochim Biophys Acta 1986; 878: 394-403; Clark et al.,Cell 1991; 65: 1043-51). The best characterized enzyme of this category is GIVA cPLA2, which presents a preference for the hydrolysis of arachidonic acid at the sn-2 position of phospholipids. This enzyme also possesses lysophospholipase and transacylase activity. The properties of the Group IV PLA2 family have been reviewed (Ghosh et al., Prog Lipid Res 2006; 45: 487-510). The structure of GIVA PLA2 shows that it is composed of a Ca2+-dependent lipid binding C2 domain and a catalytic a/b hydrolase domain. The catalytic domain is composed of a core a/b hydrolase region conserved throughout many different lipases, as well as a cap region. Ca2+ binding in this enzyme is not required for catalysis, as in the sPLA2 enzymes, but is required for translocation to the membrane surface. The enzyme possesses an unusual Ser-Asp catalytic dyad located in a deep cleft at the center of a hydrophobic funnel. The catalytic mechanism of GIVA PLA2 proceeds through a serine-acyl intermediate using Ser-228 as the nucleophilic residue. GIVA PLA2 is now generally considered to be a central enzyme mediating generation of multiple lipid mediators, including eicosanoids and potentiating pain and inflammation.

The various PLA2 types have been implicated in diverse kinds of lipid signaling and inflammatory diseases. Rheumatoid arthritis, lung inflammation, neurological disorders, such as multiple sclerosis, cardiovascular diseases, including atherosclerosis, and cancer are included among the diseases where PLA2 enzymes are involved.

For example, through the use of knockout mice deficient in the cytosolic PLA2 GIVA it has been shown that GIVA PLA2 plays a major role in inflammatory diseases (Bonventre et al., Nature 1997; 390: 622-5; Uozumi N et al., Nature 1997; 390: 618-22; Nagase et al., Nat Immunol 2000; 1: 42-6). PLA2. Moreover, a role of GIVA PLA2 in prostate cancer (Patel et al., Clin Cancer Res 2008; 14: 8070-9) and Alzheimer's disease (O Sanchez-Mejia et al., Nat Neurosci 2008; 11: 1311-18) has been proposed. In this connection, the application of cPLA2 inhibitors for the prevention and treatment of these diseases has also been proposed.

Potent and selective inhibitors of the various PLA2 enzymes have been developed and reported as agents for the treatment of the above pathophysiological situations. The regulation and inhibition of PLA2s has, for example, been reviewed by Balsinde et al. (Annu. Rev. Pharmacol. Toxicol. 1999; 39:175-89*).*

Various potent and selective PLA2 inhibitors are known in the art and have been reported in the literature. For instance, inhibitors of secreted sPLA2 are described in a review article by Reid (Curr Med Chem 2005; 12: 3011-26), while inhibitors of cytosolic cPLA2 and Ca2+-independent iPLA2 are summarized in review articles by Magrioti and Kokotos (Med Chem 2006; 5: 189-203) and Lehr (Med Chem 2006; 5: 149-61). Patents claiming inhibitors of PLA2 for the prevention and treatment of inflammation have been reviewed by Lehr (Expert Opin Ther Pat 2001; 11: 1123-36), Clark and Tam (Expert Opin Ther Pat 2004; 14: 937-50), and Magrioti and Kokotos (Expert Opin Ther Pat 2010; 20: 1-18).

### Flaviviruses

The *Flaviviridae* (also referred to as flaviviruses) are a family of viruses that infect a wide range of vertebrates and are spread through arthropods, mainly ticks and mosquitoes, or are transmitted parenterally (through blood) as well as sexually and vertically (from mother to child). The family Flaviviridae comprises three genera: Genus Flavi, Genus Hepaci, and Genus Pesti.

The genus *Flavi,* for example, includes the species Dengue Virus 1-4; West Nile Virus; Yellow Fever Virus; Tick-borne Encephalitis Virus; Japanese Encephalitis Virus; St. Louis Encephalitis Virus; Murray Valley Encephalitis Virus; Kunjin Encephalitis Virus; Rocio Encephalitis Virus; Russian Spring Summer Encephalitis Virus; Negeishi Virus; Kyasanur Forest Virus; Omsk Hemorrhagic Fever Virus; Powassan Virus; Louping Ill Virus; Rio Bravo Virus 1-7; Tyuleniy Virus; Ntaya Virus; Uganda S Virus/Zika Virus; and Modoc Virus.

The genus *Hepaci* includes the species Hepatitis C Virus and Hepatitis G Virus.

The genus *Pesti* contains viruses infecting non-human mammals, e. g. Bovine virus diarrhea 1-3.

The Flaviviruses are small (about 40 - 60 nm in diameter), enveloped, single-stranded RNA viruses with linear non-segmented genomes. The Flaviviridae genome is infectious and on average about 9.6 to 12.3 kilobase in length - it encodes around 10 genes. Viruses in this family are considered positive (+) sense because proteins are made directly from the template strand of RNA which is present in the viral capsid. Unlike cellular mRNA, the genome of flaviviruses lacks a poly-A tail.

The genomic RNA, which may have a 5' cap but no poly(A) tail, is translated as a single polyprotein that is then cleaved into at least three structural proteins and seven non-structural (NS) proteins by both viral and host proteases. The structural proteins are found at the N-terminal region of the polyprotein, while the NS proteins are located at the C-terminus. This organization allows the viruses to maximize production of structural proteins, since viral assembly requires more structural proteins to be made than NS proteins. The structural proteins are as follows: C/V2 are the capsid proteins, M/V1 are the matrix proteins and E/V3 are the envelope proteins and glycoproteins. The NS proteins include an RNA-dependent RNA polymerase (NS5), a helicase/protease (NS3), and other proteins that form part of the viral replication complex. Sequences and structures in the 5' and 3' untranslated regions (UTR) and capsid gene, including the cyclization sequences, the upstream AUG region, and the terminal 3' stem-loop, regulate translation, RNA synthesis and viral replication. Replication of Flaviviruses takes place in the cytoplasm.

Major diseases caused by the *Flaviviridae* family include: Hepatitis C Virus Infection; Dengue fever; encephalitis; and hemorrhagic fever.

An overview on flaviviruses can, for example, be found in Westaway EG, et al.,"Flaviviridae", Intervirology 1985; 24(4): 183-92; Chambers TJ and Rice CM, "Molecular biology of the flaviviruses", Microbiol Sci. 1987; 4(7): 219-23; and Knipe DM and Peter MH, eds. Fields Virology; 5th ed. Vol. 2; Philidelphia, PA: Wolters Kluwer Health, 2007; p. 1102-1291.

### Hepatitis C

Hepatitis C is an infectious disease affecting the liver, caused by the hepatitis C virus (hereinafter also referred to as HCV). Viruses are infectious agents that are found in virtually all life forms, including humans, animals, plants, fungi, and bacteria. Viruses often damage or kill the cells that they infect, causing disease in infected organisms. The difficulty in treating virus infections stems from the large number of variant viruses that can cause the same disease, as well as the inability of many drugs to disable a virus without disabling healthy cells. HCV is a small (50 nm in size), enveloped, single-stranded, positive sense RNA virus. HCV is the only known member of the *hepacivirus* genus in the family *Flaviviridae.* There are six major genotypes of HCV, which are indicated numerically (e.g., genotype 1, genotype 2, etc.).

An estimated 170 million people worldwide are infected with hepatitis C, and about 3-4 million more people per year are infected by HCV. HCV is spread by blood-to-blood contact. Hepatitis C is a strictly human disease. It cannot be contracted from or given to any other animal. Chimpanzees can be infected with the virus in the laboratory, but do not develop the disease, which has made research more difficult. No vaccine against hepatitis C is available. HCV is one of five known hepatitis viruses: A, B, C, D, and E. During the acute phase HCV infection is often asymptomatic. Most people have few, if any symptoms after the initial infection, yet the virus persists in the liver in about 85% of those infected.

HCV induces chronic infection in 50%-80% of infected persons. Approximately 50% of these do not respond to therapy. There is a very small chance of clearing the virus spontaneously in chronic HCV carriers (0.5% to 0.74% per year).

Once established, chronic infection can progress to scarring of the liver (fibrosis), and advanced scarring (cirrhosis) which is generally apparent after many years. In some cases, those with cirrhosis will go on to develop liver failure or other complications of cirrhosis, including liver cancer or life threatening esophageal varices and gastric varices. HCV is currently a leading cause of cirrhosis, a common cause of hepatocellular carcinoma, and as a result of these conditions it is the leading reason for liver transplantation.

Persistent infection can be treated with medication. Current treatment is a combination of pegylated interferon-alpha-2a or pegylated interferon-alpha-2b (brand names Pegasys or PEG-Intron) and the antiviral drug ribavirin for a period of 24 or 48 weeks, depending on hepatitis C virus genotype. Pegylated interferon-alpha-2a plus ribavirin may increase sustained virological response among patients with chronic hepatitis c as compared to pegylated interferon-alpha-2b plus ribavirin according to a systematic review of randomized controlled trials (Lagging et al., Scandinavian Journal of Infectious Diseases 2009, 41: 389-402). The treatment may be physically demanding and a substantial proportion of patients experience a panoply of side effects ranging from a 'flu-like' syndrome to severe adverse events including anemia, cardiovascular events and psychiatric problems. Fifty-one percent are cured overall. Those who develop cirrhosis or liver cancer may require a liver transplant, and the virus universally recurs after transplantation.

Treatment with the drug viramidine, which is a prodrug of ribavirin, is in phase III experimental trials against hepatitis C and other new drugs are under development like protease inhibitors (including telaprevir/VX 950), entry inhibitors (such as *SP 30* and *ITX 5061*) and polymerase inhibitors (such as *RG7128, PSI-7977* and *NM 283*). Protease inhibitor *BILN 2061* had to be discontinued due to safety problems early in the clinical testing. Some more modern new drugs that provide some support in treating HCV are *Albuferon and Zadaxin.*

Thus, although a number of methods exist or are currently under development for the treatment of HCV, many of these are complicated by the side effects the anti-viral agent(s) has/have on the patient. Additionally, drug-resistance and genotype-specific differences of HCV may limit the efficacy of these methods. As a result, a need remains to provide easily applicable methods and agents that may be used to effectively treat HCV, particularly in view of the fact that vaccination is not possible.

It is, therefore, an aim of the present invention to provide means of inhibiting infection with HCV and/or HCV replication, and of treating persistent infection with HCV and associated syndromes in patients, which treatment is preferably more effective and not as burdensome as current treatments (not to mention transplantation) and improves the lives of the patients.

### Dengue

Dengue fever, also known as breakbone fever, is an infectious tropical disease caused by the dengue virus (hereinafter also referred to as DENV). The incidence of dengue fever has increased dramatically since the 1960s, with around 50-100 million people infected yearly. Early descriptions of the condition date from 1779, and its viral cause and the transmission were elucidated in the early 20th century. Dengue has become a worldwide problem since the Second World War and is endemic in more than 110 countries.

Symptoms include fever, headache, muscle and joint pains, and a characteristic skin rash that is similar to measles. In a small proportion of cases the disease develops into the life-threatening dengue hemorrhagic fever, resulting in bleeding, low levels of blood platelets and blood plasma leakage, or into dengue shock syndrome, where dangerously low blood pressure occurs. Treatment of acute dengue is supportive, using either oral or intravenous rehydration for mild or moderate disease, and intravenous fluids and blood transfusion for more severe cases.

Dengue is transmitted by several species of mosquito within the genus *Aedes,* principally *A. aegypti.* The virus has four different types; infection with one type usually gives lifelong immunity to that type, but only short-term immunity to the others. Subsequent infection with a different type increases the risk of severe complications.

Although a number of methods exist or are currently under development for the treatment of dengue fever, there are no specific treatments for dengue fever. Moreover, many of these treatments are complicated by the side effects the anti-viral agent(s) has/have on the patient. Additionally, drug-resistance and genotype-specific differences of DENV limit the efficacy of these methods. As a result, a need remains to provide easily applicable methods and agents that may be used to effectively treat dengue, particularly in view of the fact that no approved vaccines exist. Presently, prevention solely depends on control of and protection from the bites of the mosquito that transmits it.

It is, therefore, an aim of the present invention to provide means of inhibiting infection with DENV and/or DENV replication, and of treating infection with DENV, especially persistent infections, and associated syndromes in patients, which treatment is preferably more effective and not as burdensome as current treatments (e. g. blood transfusion) and improves the lives of the patients.

### Summary and Description of the Invention

The present invention was made in view of the prior art and the needs described above, and, therefore, the object of the present invention is to provide novel alternative means for treating and/or preventing an infection with a flavivirus of the genus Flavi or the genus Hepaci.

In particular, an object of the present invention is to provide novel alternative means for treating and/or preventing an infection with HCV.

It is also an object of the present invention to provide novel alternative means for treating and/or preventing an infection with DENV.

Other objects of the present invention are to provide a pharmaceutical composition for use in the treatment or prevention of such flavivirus infections, e. g. HCV, DENV, etc., comprising at least one inhibitor of cytosolic phospholipase A2 alpha (also referred to as cPLA2α or cPLA2a inhibitor), and to provide a combinatorial composition, comprising at least one cPLA2α inhibitor and at least one further active pharmaceutical ingredient for use in the treatment or prophylaxis of an infection with a flavivirus of the genus Flavi or the genus Hepaci.

In particular, other objects of the present invention are to provide a pharmaceutical composition for use in the treatment or prevention of a HCV infection, comprising at least one cPLA2α inhibitor, and to provide a combinatorial composition, comprising at least one cPLA2α inhibitor and at least one further active pharmaceutical for treating patients infected by HCV.

These objects are solved by the subject matter of the attached claims.

These and other aspects of the present invention will become apparent upon reference to the following detailed description and definitions.

The inventors established that targeting an ubiquitous step of the viral life cycle of flaviviruses, especially flaviviruses of the genera Flavi and Hepaci, improves viral response rates and therapy success. In particular, the inventors established that targeting an ubiquitous step of the viral life cycle of all HCV genotypes improves viral response rates and therapy success. Also, the inventors established that targeting an ubiquitous step of the viral life cycle of all DENV genotypes improves viral response rates and therapy success.

The inventors showed that inhibition of cellular cytosolic Phospholipase A2 alpha (cPLA2α or cPLA2α) interferes with assembly and release of infectious progeny particles of HCV, and DENV.

The inventors also showed that RNA replication, assembly, and release of infectious virions of the vesicular stomatitis virus (VSV), a negative strand RNA virus, is not affected. These results show that cPLA2a enzyme activity is specific to assembly and release of progeny particles of flaviviruses of the genera Flavi and Hepaci, e. g. HCV and DENV, thus rendering flaviviridae of the genera Flavi and Hepaci, especially HCV and DENV susceptible to inhibition by cPLA2α inhibitors. In particular, these results show that cPLA2a enzyme activity is specific to assembly and release of progeny particles of HCV, thus rendering HCV susceptible to inhibition by cPLA2α inhibitors. The same applies *mutatis mutandis* to DENV.

The inventors also showed that virus production of HCV could be potently inhibited in a dose-dependent manner by a cPLA2α inhibitor. The same results were obtained in relation to DENV.

Similarly, the inventors showed that addition of a cPLA2α inhibitor led to a significant reduction of extracellular hepatitis C virus titers and dengue virus titers, respectively, while intracellular levels were less affected. Specifically, extracellular hepatitis C virus titers and dengue virus titers were at least 10-times lower than the intracellular virus titers. From this the inventors concluded that in HCV and DENV cPLA2α activity is responsible for assembly and release of infectious particles.

These findings were confirmed by biochemical data from fractionated lipid droplets, which data show that cPLA2α plays a role in associating hepatitis C core protein with lipid droplets. This step is known to be a critical step in virus assembly.

Finally, the inventors showed that cPLA2α activity influences the assembly and release of infectious HCV particles through governing the release of lipoproteins carrying the apolipoprotein B (ApoB) and apolipoprotein E (ApoE), which apolipoproteins are known to be essential cellular co-factors for assembly and release of infectious HCV particles. Specifically, it was shown that treatment of cells with the cPLA2a-specific inhibitor pyrrolidine-2 reduced the quantity of secreted ApoB and ApoE by more than 50%.

These surprising and unexpected results for the first time allow a therapeutic, preventive and/or curative role to be conceived for cPLA2α inhibitors in the treatment of an infection with a flavivirus of the genus Flavi or the genus Hepaci. In particular, these surprising and unexpected results for the first time allow a therapeutic, preventive and/or curative role to be conceived for cPLA2α inhibitors in the treatment of an HCV infection or DENV infection.

Accordingly, the present invention is directed to an inhibitor of cytosolic phospholipase A2 alpha (cPLA2α inhibitor) for use in the treatment or prevention of an infection with a flavivirus of the genus Flavi or the genus Hepaci.

Accordingly, the present invention is directed to an inhibitor of cytosolic phospholipase A2 alpha (cPLA2α inhibitor) for use in the treatment or prevention of an infection with hepatitis C virus (also referred to as HCV infection).

The present invention is also directed to an inhibitor of cytosolic phospholipase A2 alpha (cPLA2α inhibitor) for use in the treatment or prevention of an infection with dengue virus (also referred to as DENV infection).

By "treatment or prevention" is preferably meant a reduction or complete inhibiton of viral replication and/or infectivity in a subject to thereby cure or prevent the symptoms associated with the viral infection.

By cPLA2α inhibitor is meant any chemical or biological, natural or synthetic molecule, any composition which, whatever the mechanism, causes after administration a reduction, or even a complete inhibition, of the activity of a member of the cPLA2α protein family or the expression of a cPLA2α gene thereof. The cPLA2α inhibitor may either bind reversible or irreversible to its substrate. Reversible cPLA2α inhibitors comprise competitive inhibitors, non-competitive inhibitors, mixed-type inhibitors, uncompetitive inhibitors, slow-binding or tight-binding inhibitors, transition state analogs and multisubstrate analogs.

In an embodiment of the present invention, the cPLA2α inhibitor is an irreversible inhibitor of cPLA2α.

In another embodiment of the invention, the cPLA2α inhibitor is a reversible inhibitor of cPLA2α. Preferably, the reversible cPLA2α inhibitor is a competitive inhibitor.

Further preferred, the cPLA2α inhibitor according to the invention is a small molecule, i.e. a molecule having a molecular weight of less than about 2.5 kDa, preferably less than 800 Da, and most preferably less than 500 Da.

Examples of PLA2 inhibitors, including cPLA2α inhibitors, are presented in the review articles of Reid (Curr Med Chem 2005; 12: 3011-26), Magrioti and Kokotos (Med Chem 2006; 5: 189-203; Expert Opin Ther Pat 2010; 20: 1-18), Lehr (Med Chem 2006; 5: 149-61; Expert Opin Ther Pat 2001; 11: 1123-36), and Clark and Tam (Expert Opin Ther Pat 2004; 14: 937-50) and the numerous references cited therein.

Numerous cPLA2 inhibitors, including cPLA2α inhibitors, are known and have been reported in the art. By way of example, the following cPLA2 inhibitors can be mentioned: Pyrrolidine-2: N-{(2S,4R)-4-(Biphenyl-2-ylmethyl-isobutyl-amino)-1-[2-(2,4-difluorobenzoyl)-benzoyl]-pyrrolidin-2-ylmethyl}-3-[4-(2,4-dioxothiazolidin-5-ylidenemethyl)-phenyl]acrylamide, HCl (Calbiochem product no. 525143-500UG; Yamamoto et al., Pharmacology, 2008; 81: 301-11, DOI 10.1159/000117816); ATK (arachidonyl trifluoromethyl ketone; Ackermann et al., J. Biol. Chem., 1995; 270: 445-50), pyrrolidine-1 (Seno et al., J. Med. Chem. 2000; 43: 1041-44), MAFP (methyl arachidonyl fluorophosphonate; Lio et al., Biochim. Biophys. Acta; 1996; 1302: 55-60), ML3196 (Lehr, J. Med. Chem., 1997, 40: 2694-2705), 4-[4-[2-[2-[bis (4-chlorophenyl)methoxy]ethyl-sulphonyl]ethoxy]phenyl]-1,1-,1-trifluoro-2-butanone, BMS-229724, (Burke et al., J. Pharmacol. Exp. Ther., 2001, 298: 376-85), 3,3-dimethyl-6-(3-lauroylureido)-7-oxo-4-thia-1-azabicyclo[3,2,0]heptane-2-carboxylic acid, RSC-3388 (N[[2S,4R)-4-[([1,1'-biphenyl]-2-ylmethyl)(2-methylpropyl)amino]-1-[2-(2,4-difluorobenzoyl)benzoyl]-2-pyrrolidinyl]methyl]-3-[4-[(2,4-dioxo-5-thiazolidinylidene)methyl]phenyl]-2-propenamide; CAS 337307-06-9), indole-based cPLA2 inhibitors as described in WO 03/048122; US 2003149029; WO 2006/128142; US2006014759; WO2008/055136; WO 2008/055141; WO 2008/055146; WO 2008/055148; WO 2007/140317; US 6,630,496; US 6,350,892; McKew et al., J Med Chem 2006; 49: 135-58; Gopalsamy et al., Bioorg Med Chem Lett 2006; 16: 2978-81; Lee et al. J Med Chem 2007; 50: 1380-400; Lee et al., Bioorg Med Chem 2008; 16: 1345-58; McKew et al., J Med Chem 2008; 51: 3388-413; Marusic et al., J Neuroimmunol 2008; 204: 29-37; Chen et al., J Med Chem 2009; 52: 1156-171; Kirincich et al., Bioorg Med Chem 2009; 17: 4383-405;
heteroaryl-substituted acetone derivative cPLA2 inhibitors as described in WO 2004/069797;
1-indol-1-yl-propan-2-one cPLA2 inhibitors as described in WO 2009/040314; Ludwig et al., J Med Chem 2006; 49: 2611-20; Hess et al., Bioorg Med Chem 2007; 15: 2883-91; Fritsche et al., Bioorg Med Chem 2008; 16: 3489-500; Bovens et al., Bioorg Med Chem Lett 2009; 19: 2107-11; in particular 1-(3-(4-Octylphenoxy)-2-oxopropyl)-3-(2,2,2-trifluoroacetyl)-1H-indole-5-carboxylic acid; 3-(3-methyl-1,2,4-oxadiazol-5-yl)-1-(3-(4-octylphenoxy)-2-oxopropyl)-1H-indole-5-carboxylic acid; and 3-(3-methyl-1,2,4-oxadiazol-5-yl)-1-(2-oxo-3-(4-phenoxyphenoxy)propyl)-1H-indole-5-carboxylic acid;
2-oxoamide cPLA2 inhibitors as described in WO 03/076389; WO 2007/022443; WO 2009/009449; Kokotos et al., J Med Chem 2004; 47: 3615-28; Yaksh et al., J Pharm Exp Ther 2006; 316: 466-75; Stephens et al., J Med Chem 2006; 49: 2821-8; Six et al. , J Med Chem 2007; 50: 4222-35; Antonopoulou et al., Bioorg Med Chem 2008; 16: 10257-69; Barbayianni et al., Bioorg Med Chem 2009; 17: 4833-43; Burke et al., J Am Chem Soc 2009; 131: 8083-91; in particular cPLA2α inhibitors 3-(3-methyl-1,2,4-oxadiazol-5-yl)-1-(2-oxo-3-(4-phenoxyphenoxy)propyl)-1H-indole-5-carboxylic acid; 4-(2-oxohexadecanamido)butanoic acid (AX006), 4-(2-oxododecanamido)octanoic acid (AX007), 4-(2-oxohexadecanamido)butanoic acid ethyl ester (AX048), 4,4-[(2-oxohexadecanamido)-2-methylpropyl]butanoic acid (AX059), and 2-(2-oxohexadecanamido) acetic acid methyl ester (AX115);
substituted isothiazolone cPLA2 inhibitors as described in WO 2007/001932;
alpha-amino, -thio, -oxo substituted ketone cPLA2 inhibitors as described in US 2002/0037875;
thiazolidinedione cPLA2 inhibitors as described in Seno et al., J Med Chem 2000; 43: 1041-4; Eno et al., Bioorg Med Chem Lett 2001; 11: 587-90; Ono et al., Biochem J 2002; 363: 727-35; and Tai et al., Inflamm Res; published online 5 August 2009, DOI 10.1007/s00011-009-0069-8;
and those described in WO 2007/118996 and WO 03/101487.

The cPLA2α inhibitor can be selected from N-{(2S,4R)-4-(Biphenyl-2-ylmethyl-isobutyl-amino)-1-[2-(2,4-difluorobenzoyl)-benzoyl]-pyrrolidin-2-ylmethyl}-3-[4-(2,4-dioxothiazolidin-5-ylidenemethyl)-phenyl]acrylamide (pyrrolidine-2); arachidonyl trifluoromethyl ketone (ATK); methyl arachidonyl fluorophosphonate (MAFP) ; 4-[4-[2-[2-[bis(4-chlorophenyl) methoxy]ethyl-sulphonyl]ethoxy]phenyl]-1,1-,1-trifluoro-2-butanone; 3,3-dimethyl-6-(3-lauroylureido)-7-oxo-4-thia-1-azabicyclo[3,2,0]heptane-2-carboxylic acid; (N[[2S,4R)-4-[([1,1'-biphenyl]-2-ylmethyl)(2-methylpropyl)amino]-1-[2-(2,4-difluorobenzoyl)benzoyl]-2-pyrrolidinyl]methyl]-3-[4-[(2,4-dioxo-5-thiazolidinylidene)methyl]phenyl]-2-propenamide (RSC-3388); 1-(3-(4-octylphenoxy)-2-oxopropyl)-3-(2,2,2-trifluoroacetyl)-1H-indole-5-carboxylic acid; 3-(3-methyl-1,2,4-oxadiazol-5-yl)-1-(3-(4-octylphenoxy)-2-oxopropyl)-1H-indole-5-carboxylic acid; 3-(3-methyl-1,2,4-oxadiazol-5-yl)-1-(2-oxo-3-(4-phenoxyphenoxy)propyl)-1H-indole-5-carboxylic acid; 4-(2-oxohexadecanamido)butanoic acid (AX006), 4-(2-oxododecanamido)octanoic acid (AX007), 4-(2-oxohexadecanamido)butanoic acid ethyl ester (AX048), 4,4-[(2-oxohexadecanamido)-2-methylpropyl]butanoic acid (AX059), and 2-(2-oxohexadecanamido) acetic acid methyl ester (AX115).

Especially preferred according to the invention, the cPLA2α inhibitor is pyrrolidine-2.

According to the invention, it is also possible to use a combination of PLA2 inhibitors (2 or more), preferably a combination of 2 or more cPLA2 inhibitors, wherein at least one of the PLA2 inhibitors is a cPLA2α inhibitor, i. e., for example, one cPLA2α inhibitor in combination with one or more PLA2 inhibitor(s) selected from sPLA2, cPLA2, iPLA2 or LpPLA2 inhibitors.

The invention is also directed to the use of at least one cPLA2α inhibitor for the preparation of a medicament intended for the treatment of an infection with a flavivirus of the genus Flavi or the genus Hepaci, e. g. HCV infection or DENV infection. In particular, the invention is also directed to the use of at least one cPLA2α inhibitor for the preparation of a medicament intended for the treatment of a HCV infection, or a DENV infection. As detailed above, cPLA2α inhibitors have been widely described in the literature and any one of those can be employed.

The term "at least one" as used herein is intended to mean one, 2, or more of the respective item or subject.

Moreover, the invention is also directed to a pharmaceutical composition for use in the treatment or prevention of an infection with a flavivirus of the genus Flavi or the genus Hepaci, e. g. HCV infection or DENV infection, which pharmaceutical composition comprises one or more cPLA2α inhibitor(s), and, optionally, at least one pharmaceutically acceptable carrier(s) and/or at least one customary excipient. In particular, the invention is also directed to a pharmaceutical composition for use in the treatment or prevention of a HCV infection, or DENV infection, which pharmaceutical composition comprises one or more cPLA2α inhibitor(s), and, optionally, at least one pharmaceutically acceptable carrier(s) and/or at least one customary excipient.

Carrier substances are, for example, cyclodextrins such as hydroxypropyl β-cyclodextrin, micelles or liposomes, excipients and/or adjuvants. Pharmaceutical compositions may additionally comprise, for example, one or more of water, buffers such as, *e*.*g*., neutral buffered saline or phosphate buffered saline, ethanol, mineral oil, vegetable oil, dimethylsulfoxide, carbohydrates such as *e.g.,* glucose, mannose, sucrose or dextrans, mannitol, proteins, adjuvants, polypeptides or amino acids such as glycine, antioxidants, chelating agents such as EDTA or glutathione and/or preservatives. Furthermore, one or more other active ingredients may, but need not, be included in the pharmaceutical compositions provided herein. For instance, the compounds of the invention may advantageously be employed in combination with an antibiotic, anti-fungal, or anti-viral agent, an anti-histamine, a non-steroidal anti-inflammatory drug, a disease modifying anti-rheumatic drug, a cytostatic drug, a drug with smooth muscle activity modulatory activity or mixtures of the aforementioned.

Pharmaceutical compositions may be formulated for any appropriate route of administration, including, for example, topical such as, *e.g.,* transdermal or ocular, oral, buccal, nasal, vaginal, rectal or parenteral administration. The term parenteral as used herein includes subcutaneous, intradermal, intravascular such as, *e.g.,* intravenous, intramuscular, spinal, intracranial, intrathecal, intraocular, periocular, intraorbital, intrasynovial and intraperitoneal injection, as well as any similar injection or infusion technique. In certain embodiments, compositions in a form suitable for oral use are preferred. Such forms include, for example, tablets, troches, lozenges, aqueous or oily suspensions, dispersible powders or granules, emulsion, hard or soft capsules, or syrups or elixirs. Within yet other embodiments, compositions provided herein may be formulated as a lyophilizate.

Compositions intended for oral use may further comprise one or more components such as sweetening agents, flavoring agents, coloring agents and/or preserving agents in order to provide appealing and palatable preparations. Tablets contain the active ingredient in admixture with customary (physiologically acceptable) excipients that are suitable for the manufacture of tablets.

Customary excipients include, for example, inert diluents such as, *e.g.,* calcium carbonate, sodium carbonate, lactose, calcium phosphate or sodium phosphate, granulating and disintegrating agents such as, *e.g.,* corn starch or alginic acid, binding agents such as, *e.g.,* starch, gelatin or acacia, and lubricating agents such as, *e.g.,* magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monosterate or glyceryl distearate may be employed.

The pharmaceutical composition of the invention may comprise at least one cPLA2α inhibitor. The pharmaceutical composition may also comprise 2 or more cPLA2α inhibitors.

Preferably, the pharmaceutical composition of the invention comprises at least one cPLA2α inhibitor, which is selected from pyrrolidine-2; RSC-3388; ATK; MAFP; 4-[4-[2-[2-[bis(4-chlorophenyl) methoxy]ethyl-sulphonyl]ethoxy]phenyl]-1,1-,1-trifluoro-2-butanone; 3,3-dimethyl-6-(3-lauroylureido)-7-oxo-4-thia-1-azabicyclo[3,2,0]-heptane-2-carboxylic acid; 1-(3-(4-octylphenoxy)-2-oxopropyl)-3-(2,2,2-trifluoroacetyl)-1H-indole-5-carboxylic acid; 3-(3-methyl-1,2,4-oxadiazol-5-yl)-1-(3-(4-octylphenoxy)-2-oxopropyl)-1H-indole-5-carboxylic acid; 3-(3-methyl-1,2,4-oxadiazol-5-yl)-1-(2-oxo-3-(4-phenoxyphenoxy)propyl)-1H-indole-5-carboxylic acid; AX006; AX007; AX048; AX059 or AX115. Especially preferred according to the invention, the pharmaceutical composition contains the cPLA2α inhibitor pyrrolidine-2.

For the treatment or prevention of an infection with a flavivirus of the genus Flavi or the genus Hepaci, e. g. HCV infection or DENV infection, the dose of the biologically active compound according to the invention may vary within wide limits and may be adjusted to individual requirements. Active compounds according to the present invention are generally administered in a therapeutically effective amount. Preferred doses range from about 0.1 mg to about 140 mg per kilogram of body weight per day, and/or about 0.5 mg to about 7 g per patient per day. The daily dose may be administered as a single dose or in a plurality of doses. The amount of active ingredient that may be combined with the carrier materials to produce a single dosage form will vary depending upon the host treated and the particular mode of administration. Dosage unit forms will generally contain between from about 1 mg to about 500 mg of an active ingredient.

According to the invention, the cPLA2α inhibitor(s) can be used in the pharmaceutical preparation in a quantity comprised between 0.01 mg and 2 g, preferably from 1 mg to 1 g, very preferably from 10 mg to 500 mg.

More particularly, the cPLA2α inhibitor(s) such as, e. g. pyrrolidine-2, RSC-3388 or ATK, can in particular be administered in doses comprised between 0.1 mg/kg and 500 mg/kg, preferably 1 mg/kg and 100 mg/kg, very preferably 10 mg/kg and 50 mg/kg.

It will be understood, however, that the specific dose level for any particular patient will depend upon a variety of factors including the activity of the specific compound employed, the age, body weight, general health, sex, diet, time of administration, route of administration, and rate of excretion, drug combination, i.e. other drugs being used to treat the patient, and the severity of the particular disease undergoing therapy.

The invention is also related to a combination preparation containing at least one cPLA2α inhibitor and at least one further active pharmaceutical ingredient for the treatment or prophylaxis of an infection with a flavivirus of the genus Flavi or the genus Hepaci, e. g. HCV infection or DENV infection. In particular, the invention is also related to a combination preparation containing at least one cPLA2α inhibitor and at least one further active pharmaceutical ingredient for the treatment or prophylaxis of a HCV infection, or DENV infection.

Preferably, in the combination preparation of the invention the further active pharmaceutical ingredient is selected from pegylated interferon-alpha-2a, pegylated interferon-alpha-2b, ribavirin, viramidine, telaprevir/VX 950, *SP 30, ITX 5061, RG7128, PSI-7977, NM 283, Albuferon and*/*or Zadaxin.*

Most preferred is a combination of at least one cPLA2α inhibitor and at least one of pegylated interferon-alpha-2a, pegylated interferon-alpha-2b, ribavirin and viramidine.

Preferably, the at least one cPLA2α inhibitor contained in the combination preparation of the invention is selected from pyrrolidine-2; RSC-3388; ATK; MAFP; 4-[4-[2-[2-[bis (4-chlorophenyl) methoxy]ethyl-sulphonyl]ethoxy]phenyl]-1,1-,1-trifluoro-2-butanone; 3,3-dimethyl-6-(3-lauroylureido)-7-oxo-4-thia-1-azabicyclo[3,2,0]- heptane-2-carboxylic acid; 1-(3-(4-octylphenoxy)-2-oxopropyl)-3-(2,2,2-trifluoroacetyl)-1H-indole-5-carboxylic acid; 3-(3-methyl-1,2,4-oxadiazol-5-yl)-1-(3-(4-octylphenoxy)-2-oxopropyl)-1H-indole-5-carboxylic acid; 3-(3-methyl-1,2,4-oxadiazol-5-yl)-1-(2-oxo-3-(4-phenoxyphenoxy)propyl)-1H-indole-5-carboxylic acid; AX006, AX007, AX048, AX059 or AX115. The combination preparation may also contain more than one cPLA2α inhibitor and additionally other active pharmaceutical ingredients.

Preferably, cPLA2α inhibitors used for the treatment of an infection with a flavivirus of the genus Flavi or the genus Hepaci, e. g. HCV infection or DENV infection, will have certain pharmacological properties. In particular, cPLA2α inhibitors used for the treatment of a HCV infection, or DENV infection, will have certain pharmacological properties. Such properties include, but are not limited to oral bioavailability, such that the preferred oral dosage forms discussed above can provide therapeutically effective levels of the compound *in vivo.*

The cPLA2α inhibitors are preferably administered to a patient orally or parenterally, and are present within at least one body fluid or tissue of the patient.

Accordingly, also provided are methods for preventing or treating patients suffering from an infection with a flavivirus of the genus Flavi or the genus Hepaci, e. g. HCV infection or DENV infection. In particular, provided are methods for preventing or treating patients suffering from a HCV infection, or DENV infection.

As used herein, the term "treatment" encompasses both disease-modifying treatment and symptomatic treatment, either of which may be prophylactic, *i.e.,* before the onset of symptoms, in order to prevent, delay or reduce the severity of symptoms, or therapeutic, *i.e.,* after the onset of symptoms, in order to reduce the severity and/or duration of symptoms.

Preferably, the method of preventing or treating an infection with a flavivirus of the genus Flavi or the genus Hepaci, e . g. a HCV infection or a DENV infection, comprises administering to a subject in need thereof an effective amount of at least one cPLA2α inhibitor. More preferably, the method of preventing or treating a HCV infection, or a DENV infection, comprises administering to a subject in need thereof an effective amount of at least one cPLA2α inhibitor.

In a preferred method of preventing or treating an infection with a flavivirus of the genus Flavi or the genus Hepaci, e. g. a HCV infection or DENV infection, the method comprises administration of at least one of the cPLA2α inhibitors detailed above in relation to cPLA2α inhibitors for use in the prevention or treatment of an infection with a flavivirus of the genus Flavi or the genus Hepaci. In a more preferred method of preventing or treating a HCV infection, or DENV infection, the method comprises administration of at least one of the cPLA2α inhibitors detailed above in relation to cPLA2α inhibitors for use in the prevention or treatment of HCV infection, or DENV infection. More particularly, the method preferably comprises administration of pyrrolidine-2.

The method of preventing or treating an infection with a flavivirus of the genus Flavi or the genus Hepaci, e. g. a HCV infection or a DENV infection, may, moreover, be characterized in that the cPLA2α inhibitor is intended to be administered by oral route, by aerosol route or by injection. In particular, the method of preventing or treating a HCV infection, or a DENV infection, may, moreover, be characterized in that the cPLA2α inhibitor is intended to be administered by oral route, by aerosol route or by injection.

It is especially preferred to combine the preferred embodiments of the present invention in any possible manner.

### Brief Description of the Figures

**Figure 1****:** Representation of the structure of the chemical structure of pyrrolidine-2.
**Figure 2****:** Assay set up for assessing inhibition of virus production in a dose dependent manner by the cPLA2α inhibitor pyrrolidine-2.
**Figure 3****: A:** RNA replication of HCV in the presence of varying concentrations of the cPLA2α inhibitor pyrrolidine-2. **B:** Release of infectious HCV particles in the presence of varying concentrations of the cPLA2α inhibitor pyrrolidine-2.
**Figure 4****: A:** Assay set up for testing effect of the cPLA2α inhibitor pyrrolidine-2 on viral entry. **B:** Graph showing the viral entry in cells in the absence of cPLA2α inhibitor pyrrolidine-2 compared to cells treated with 20 *µ*m pyrrolidine-2.
**Figure 5****:** Cytotoxic effect of cPLA2α inhibitor treatment (20 *µ*m pyrrolidine-2) compared to treatment with DMSO (left bar).
**Figure 6****:** Assay set up for determining influence of cPLA2α inhibitor pyrrolidine-2 on assembly and release of HCV
**Figure 7****:** Comparison of extracellular and intracellular virus titer in both untreated cells (-) and cells treated with 20 *µ*m pyrrolidine-2 (+).
**Figure 8****:** Effect of cPLA2α inhibitor pyrrolidine-2 on HCV core protein association with lipid droplets. ADRP (*Adipose Differentiation Related Protein*): Lipid droplet marker protein; Calreticulin: Marker protein for endoplasmic reticulum; Golgi Matrix Protein: Marker protein for Golgi. **A:** Analysis of lipid droplet fractions by Core ELISA. **B:** Analysis of total cell lysates by Western Blot. **C:** Analysis of lipid droplet fractions by Western Blot.
**Figure 9****:** Effect of cPLA2α inhibitor pyrrolidine-2 on secretion of apolipoproteins B and E. Analysis by apolipoprotein ELISA. Graph showing a comparison between both untreated Mock- and Jc1-transfected cells (-) and treated Mock- and Jc1-transfected cells (+; 20 *µ*m pyrrolidine-2).

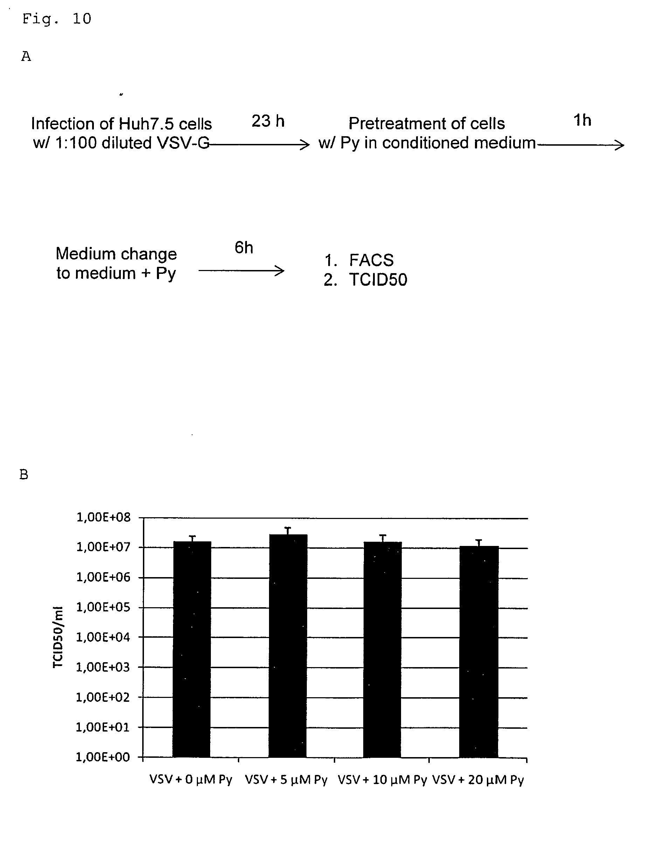

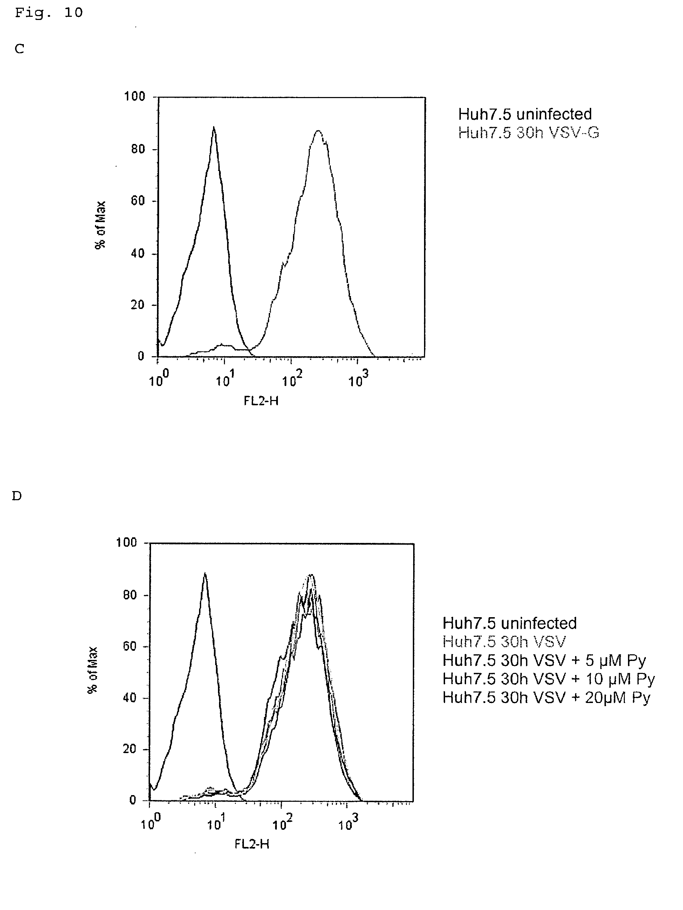

**Figure 10****:** Effect of cPLA2α inhibitor pyrrolidine-2 on production of infectious vesicular stomatitis virus (VSV) particles. **A:** Assay set up for testing effect of the cPLA2α inhibitor pyrrolidine-2 on production of VSV. **B:** TCID50 graph showing effect at various inhibitor concentrations. **C:** Analysis of uninfected (left graph) and infected untreated cells by FACS. **D:** Analysis of uninfected (left graph) and infected untreated and treated cells by FACS.
**Figure 11****: A:** RNA replication of DENV in the presence of the cPLA2α inhibitor pyrrolidine-2. **B:** Release of infectious DENV particles in the presence of the cPLA2α inhibitor pyrrolidine-2.

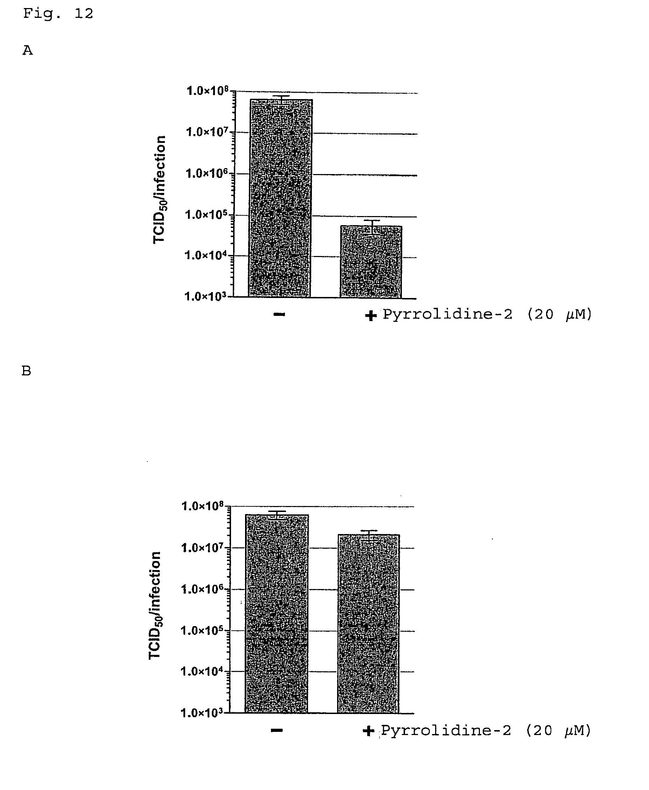

**Figure 12****: A:** Effect of cPLA2α inhibitor pyrrolidine-2 on infectivity of released DENV particles. Analysis by limiting dilution titration assay. Graph showing a comparison between both untreated Huh-7.5 cells transfected with dengue virus DNA (-) and treated Huh-7.5 cells transfected with dengue virus DNA (+; 20 *µ*m pyrrolidine-2). **B:** Effect of cPLA2α inhibitor pyrrolidine-2 on infectivity of released DENV particles that were produced in the absence of the inhibitor. Analysis by limiting dilution titration assay. Graph showing a comparison between both untreated DENV particles (-) and treated DENV particles (+; 20 *µ*m pyrrolidine-2).

The present invention is now further illustrated by the following examples, which are not construed to limit the broad aspects of the invention disclosed herein.

### Examples

### Example 1: Inhibition of HCV production

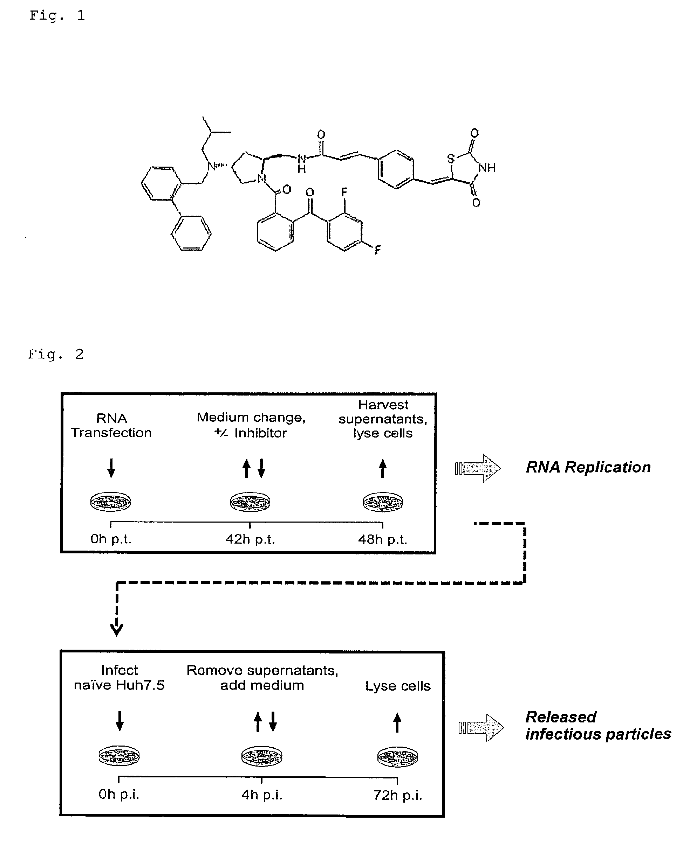

This example employs a standard bioassay for determining the effect of the cPLA2α inhibitor pyrrolidine-2 on the production of Jc1-transfected cells. The general assay set up is shown in **Fig. 2****.** The assay was performed according to standard protocols and materials were purchased from commercial suppliers and used according to respective manuals supplied therewith. Pyrrolidine-2 was added to the cells 42 h after transfection at 42 the indicated final concentrations of 0, 5, 10 and 20 *µ*M.

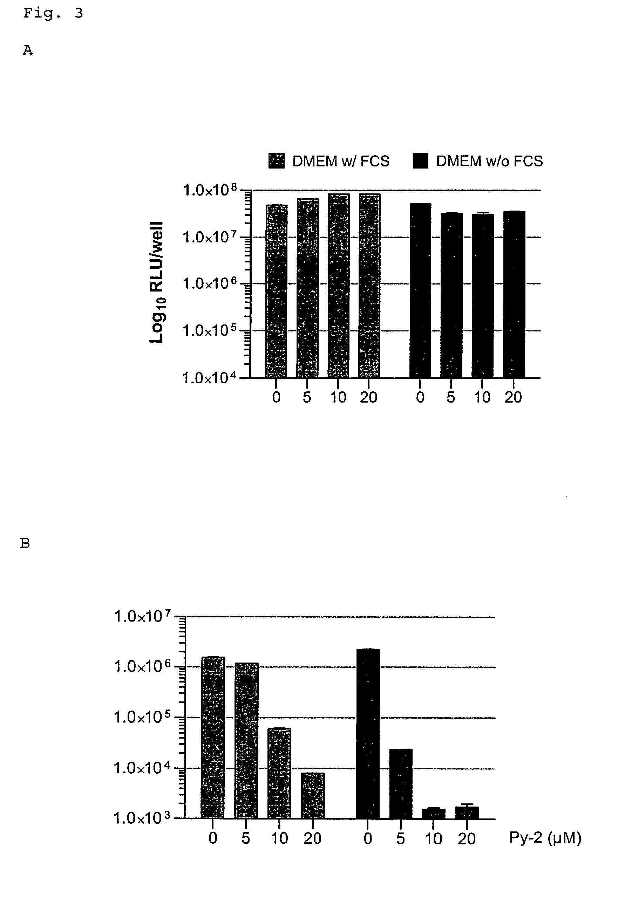

The results are presented in **Fig. 3A** and **3B****.** These results show that RNA replication was not affected by pyrrolidine-2 (Fig. 3A), while release of infectious virus particles was potently inhibited in a dose-dependent manner (Fig. 3B). Thus, the results indicate that inhibition of cPLA2 significantly lowers virus production.

### Example 2: Virus entry and Cytotoxicity

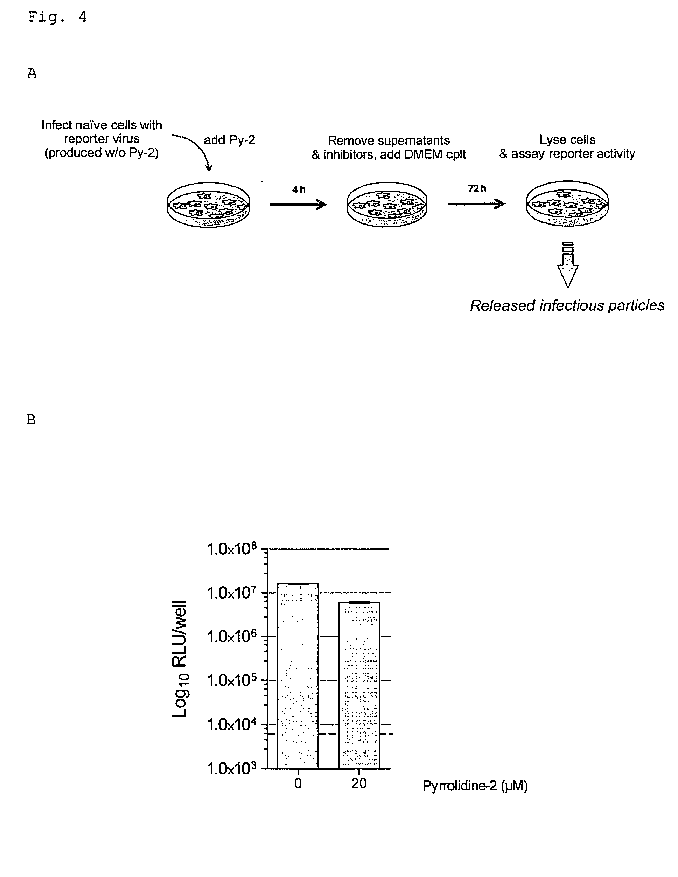

This example shows that pyrrolidine-2 has no influence on virus entry and cell viability. The general assay set up is shown in **Fig. 4A****.** The assay was performed according to standard protocols and materials were purchased from commercial suppliers and used according to respective manuals supplied therewith.

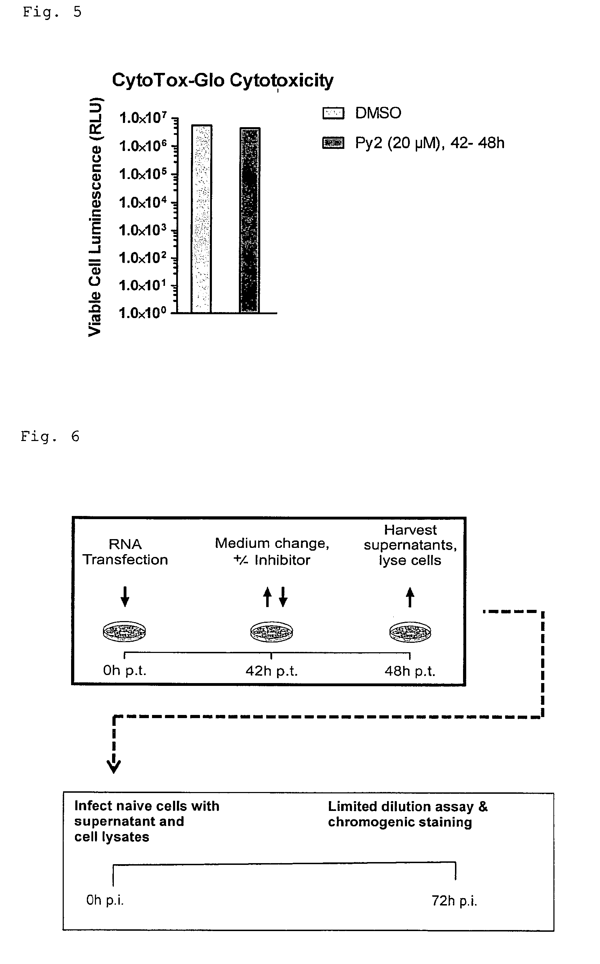

The results are shown in **Fig. 4B** and **Fig. 5****,** respectively. **Fig. 4B** shows that pyrrolidine-2 does not influence viral entry. From **Fig. 5** can be taken that pyrrolidine-2 did not show any cytotoxicity.

### Example 3: Virus assembly and release

This example shows that pyrrolidine-2 is essential for HCV assembly and release. The general assay set up is shown in **Fig. 6****.** The assay was performed according to standard protocols and materials were purchased from commercial suppliers and used according to respective manuals supplied therewith.

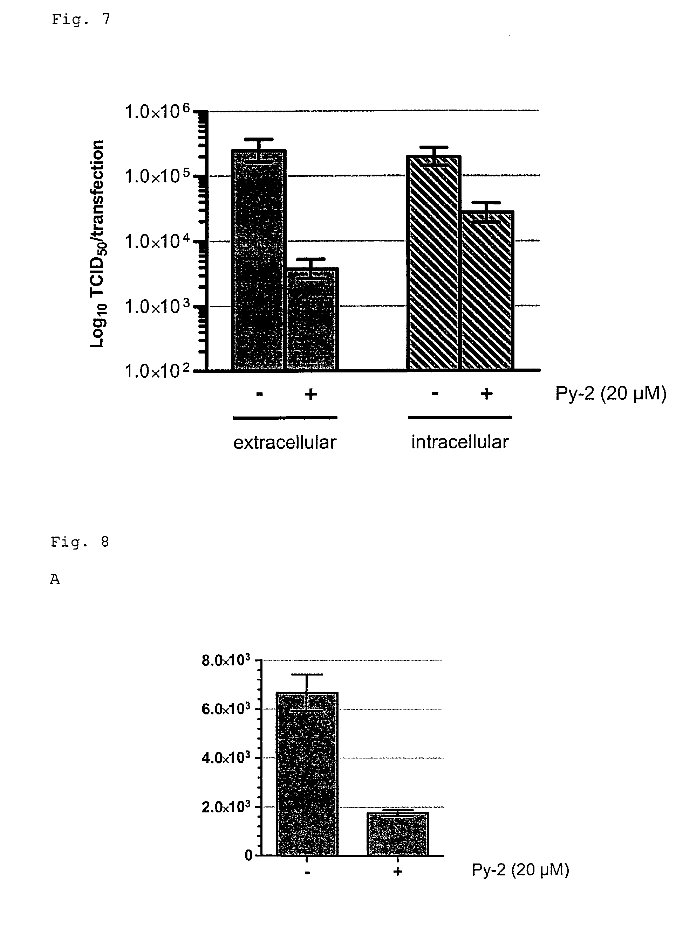

The result is shown in **Fig. 7****.** Pyrrolidine-2 led to a 100-fold reduction of extracellular virus titers. In contrast, intracellular virus titers were essentially not affected. The intracellular virus titer was only about 10-fold lowered. As a result, cPLA2α activity is essential for assembly and release of infectious virus particles.

### Example 4: HCV core protein association with lipid droplets

This example shows that pyrrolidine-2 influences the critical virus assembly step of associating HCV core protein with lipid droplets. Standard ELISA and Western Blot protocols were used for the analysis of lipid droplet fractions of untreated cells and cells treated with 20 *µ*m pyrrolidine-2.

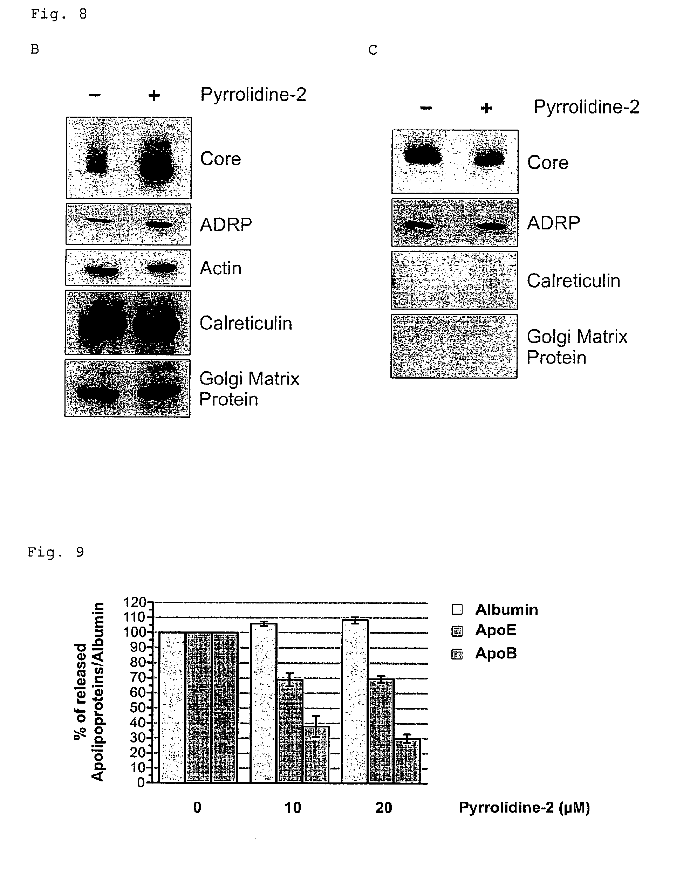

The results are shown in **Fig. 8A-C.** Pyrrolidine-2 reduced the quantity of lipid droplet associated core protein. Thus, pyrrolidine-2 suppresses an early step of HCV assembly prior to virus budding. As a result, enveloped core proteins are considerably less abundant within virus infected cells that have been treated with pyrrolidine-2.

### Example 5: Secretion of apolipoproteins

This example shows that pyrrolidine-2 affects the secretion of apolipoproteins B (ApoB) and E (ApoE). A standard ELISA protocol was employed for assessing the relevance of cPLA2α activity for release of lipoproteins carrying ApoB and ApoE.

The result is shown in **Fig. 9****.** Treatment of cells with Pyrrolidine-2 reduced the quantity of secreted ApoB and ApoE by more than 50%. These result suggests that cPLA2 activity is involved in assembly of infectious HCV particles, presumably through participating in the formation of lipoproteins.

The features of the present invention disclosed in the specification, the claims and/or the drawings may both separately and in any combination thereof be material for realizing the invention in various forms thereof. especially preferred are combinations of preferred embodiments of the invention.

### Example 6: Inhibition of DENV production

This example employs a standard bioassay for determining the effect of the cPLA2α inhibitor pyrrolidine-2 on the production of Huh-7.5 cells transfected with dengue virus RNA DV-R2A. The general assay set up is similar to that shown in **Fig. 2****.** The assay was performed according to standard protocols and materials were purchased from commercial suppliers and used according to respective manuals supplied therewith. Pyrrolidine-2 was added to the cells 42 h after transfection at the indicated final concentrations of 0, 5, 10 and 20 *µ*M.

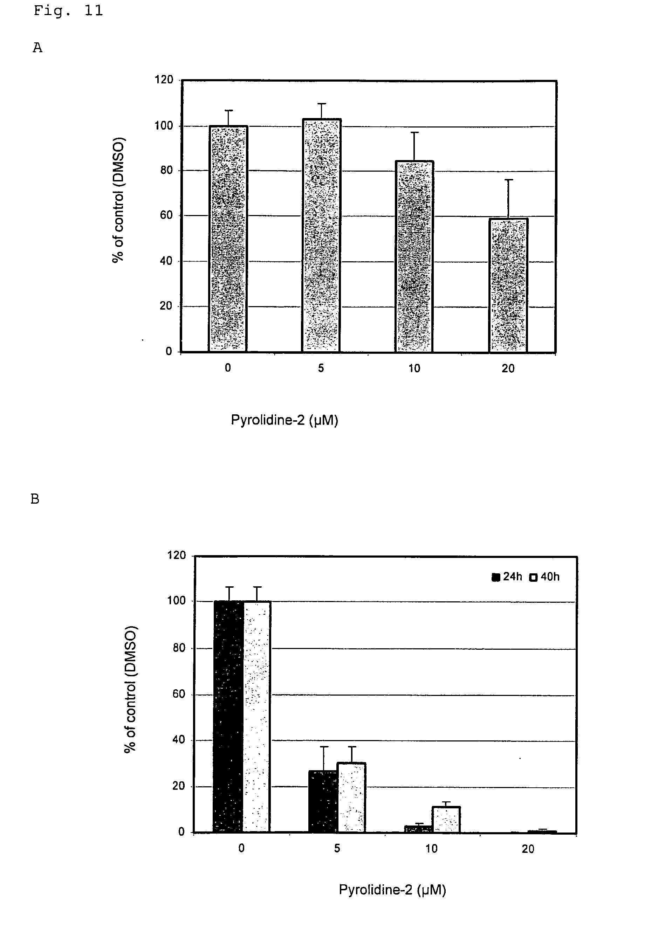

The results are presented in **Fig. 11A** and **11B****.** These results show that RNA replication was only slightly affected by pyrrolidine-2 (Fig. 11A), while release of infectious virus particles was potently inhibited in a dose-dependent manner (Fig. 11B). Thus, the results indicate that inhibition of cPLA2 significantly lowers virus production.

### Example 7: Infectivity of DENV

This example shows that pyrrolidine-2 reduces the infectivity of released particles. A standard limiting dilution titration assay was employed for assessing the relevance of cPLA2α activity for release of infectious DENV particles.

The result is shown in **Fig. 12A****.** Treatment of cells with Pyrrolidine-2 reduced the quantity of released infectious DENV particles by more than 3 orders of magnitude. Pyrrolidine-2 does not inactivate released infectious particles (Fig. 12B). These results suggest that cPLA2 is a dengue virus assembly/release factor.

## Claims

1. An inhibitor of cytosolic phospholipase A2 alpha (cPLA2α inhibitor) for use in the treatment or prevention of an infection with a flavivirus of the genus Flavi or the genus Hepaci.

2. The cPLA2α inhibitor for use as in Claim 1, wherein the cPLA2α inhibitor is selected from N-{(2S,4R)-4-(Biphenyl-2-ylmethyl-isobutyl-amino)-1-[2-(2,4-difluorobenzoyl)-benzoyl]-pyrrolidin-2-ylmethyl}-3-[4-(2,4-dioxothiazolidin-5-ylidenemethyl)-phenyl]acrylamide (pyrrolidine-2); arachidonyl trifluoromethyl ketone (ATK); methyl arachidonyl fluorophosphonate (MAFP); 4-[4-[2-[2-[bis(4-chlorophenyl) methoxy]ethyl-sulphonyl]ethoxy]phenyl]-1,1-,1-trifluoro-2-butanone; 3,3-dimethyl-6-(3-lauroylureido)-7-oxo-4-thia-1-azabicyclo[3,2,0]heptane-2-carboxylic acid; (N[[2S,4R)-4-[([1,1'-biphenyl]-2-ylmethyl)(2-methylpropyl)amino]-1-[2-(2,4-difluorobenzoyl)benzoyl]-2-pyrrolidinyl]methyl]-3-[4-[(2,4-dioxo-5-thiazolidinylidene)methyl]phenyl]-2-propenamide (RSC-3388); 1-(3-(4-octylphenoxy)-2-oxopropyl)-3-(2,2,2-trifluoroacetyl)-1H-indole-5-carboxylic acid; 3-(3-methyl-1,2,4-oxadiazol-5-yl)-1-(3-(4-octylphenoxy)-2-oxopropyl)-1H-indole-5-carboxylic acid; 3-(3-methyl-1,2,4-oxadiazol-5-yl)-1-(2-oxo-3-(4-phenoxyphenoxy)propyl)-1H-indole-5-carboxylic acid; 4-(2-oxohexadecanamido)butanoic acid (AX006), 4-(2-oxododecanamido)octanoic acid (AX007), 4-(2-oxohexadecanamido)butanoic acid ethyl ester (AX048), 4,4-[(2-oxohexadecanamido)-2-methylpropyl]butanoic acid (AX059), and 2-(2-oxohexadecanamido) acetic acid methyl ester (AX115).

3. The cPLA2α inhibitor for use as in Claim 1 or 2, wherein the cPLA2α inhibitor is N-{(2S,4R)-4-(Biphenyl-2-ylmethyl-isobutyl-amino)-1-[2-(2,4-difluorobenzoyl)-benzoyl]-pyrrolidin-2-ylmethyl}-3-[4-(2,4-dioxothiazolidin-5-ylidenemethyl)-phenyl]acrylamide (pyrrolidine-2).

4. A pharmaceutical composition for use in the treatment or prevention of infection with a flavivirus of the genus Flavi or the genus Hepaci, which pharmaceutical composition comprises one or more cPLA2α inhibitor(s), and, optionally, at least one pharmaceutically acceptable carrier(s) and/or at least one customary excipient.

5. A combination preparation for use in the treatment or prophylaxis of an infection with a flavivirus of the genus Flavi or the genus Hepaci containing at least one cPLA2α inhibitor and at least one further active pharmaceutical ingredient.

6. The combination preparation for use as in Claim 5, wherein the further active pharmaceutical ingredient is selected from pegylated interferon-alpha-2a, pegylated interferon-alpha-2b, ribavirin, viramidine, telaprevir/VX 950, SP 30, ITX 5061, RG7128, PSI-7977, NM 283, Albuferon and/or Zadaxin.

7. Use of an inhibitor of cytosolic phospholipase A2 alpha for the preparation of a medicament for preventing or treating an infection with a flavivirus of the genus Flavi or the genus Hepaci.

## Patentansprüche

1. Inhibitor der zytosolischen Phospholipase A2 alpha (cPLA2α Inhibitor) zur Verwendung bei der Behandlung oder Prävention einer Infektion mit einem Flavivirus der Gattung Flavi oder der Gattung Hepaci.

2. Der cPLA2α Inhibitor zur Verwendung wie in Anspruch 1, wobei der cPLA2α Inhibitor ausgewählt ist aus N-{(2S,4R)-4-(Biphenyl-2-ylmethyl-isobutyl-amino)-1-[2-(2,4-difluorobenzoyl)-benzoyl]-pyrrolidin-2-ylmethyl}-3-[4-(2,4-dioxothiazolidin-5-ylidenmethyl)-phenyl]acrylamid (Pyrrolidin-2); Arachidonyltrifluormethylketon (ATK); Methylarachidonylfluorphosphonat (MAFP); 4-[4-[2-[2-[Bis(4-chlorphenyl) methoxy]ethyl-sulphonyl]ethoxy]phenyl]-1,1-,1-trifluor-2-butanon; 3,3-Dimethyl-6-(3-lauroylureido)-7-oxo-4-thia-1-azabicyclo[3,2,0]heptan-2-carboxylsäure; (N[[2S,4R)-4-[([1,1'-Biphenyl]-2-ylmethyl)(2-methylpropyl)amino]-1-[2-(2,4-difluorbenzoyl)benzoyl]-2-pyrrolidinyl]methyl]-3-[4-[(2,4-dioxo-5-thiazolidinyliden)methyl]phenyl]-2-propenamid (RSC-3388); 1-(3-(4-Octylphenoxy)-2-oxopropyl)-3-(2,2,2-trifluoracetyl)-1H-indol-5-carboxylsäure; 3-(3-Methyl-1,2,4-oxadiazol-5-yl)-1-(3-(4-octylphenoxy)-2-oxopropyl)-1H-indol-5-carboxylsäure; 3-(3-Methyl-1,2,4-oxadiazol-5-yl)-1-(2-oxo-3-(4-phenoxyphenoxy)propyl)-1H-indol-5-carboxylsäure; 4-(2-Oxohexadecanamido)butansäure (AX006), 4-(2-Oxododecanamido)octansäure (AX007), 4-(2-Oxohexadecanamido)butansäureethylester (AX048), 4,4-[(2-Oxohexadecanamido)-2-methylpropyl]butansäure (AX059), und 2-(2-Oxohexadecanamido)-essigsäuremethylester (AX115).

3. Der cPLA2α Inhibitor zur Verwendung wie in Anspruch 1 oder 2, wobei der cPLA2α Inhibitor N-{(2S,4R)-4-(Biphenyl-2-ylmethyl-isobutyl-amino)-1-[2-(2,4-difluorbenzoyl)-benzoyl]-pyrrolidin-2-ylmethyl}-3-[4-(2,4-dioxothiazolidin-5-ylidenmethyl)-phenyl]acrylamid (Pyrrolidin-2) ist.

4. Pharmazeutische Zusammensetzung zur Verwendung bei der Behandlung oder Prävention einer Infektion mit einem Flavivirus der Gattung Flavi oder der Gattung Hepaci, wobei die pharmazeutische Zusammensetzung einen oder mehrere cPLA2α Inhibitor(en), und gegebenenfalls mindestens einen pharmazeutisch akzeptablen Träger und / oder mindestens einen üblichen Hilfsstoff enthält.

5. Kombinationspräparat zur Verwendung bei der Behandlung oder Prävention einer Infektion mit einem Flavivirus der Gattung Flavi oder der Gattung Hepaci, welches mindestens einen cPLA2α Inhibitor und mindestens einen weiteren pharmazeutisch aktiven Wirkstoff enthält.

6. Das Kombinationspräparat zur Verwendung wie in Anspruch 5, wobei der weitere pharmazeutisch aktive Wirkstoff ausgewählt ist aus pegyliertem Interferon-alpha-2a, pegyliertem Interferon-alpha-2b, Ribavirin, Viramidin, Telaprevir/VX 950, SP 30, ITX 5061, RG7128, PSI-7977, NM 283, Albuferon und / oder Zadaxin.

7. Verwendung eines Inhibitors der zytosolischen Phospholipase A2 alpha für die Herstellung eines Medikaments zur Prävention oder Behandlung einer Infektion mit einem Flavivirus der Gattung Flavi oder der Gattung Hepaci.

## Revendications

1. Inhibiteur de l'alpha phospholipase A2 cytosolique (inhibiteur de cPLA2α) pour une utilisation dans le traitement ou la prévention d'une infection par un flavivirus du genre Flavi ou du genre Hepaci.

2. Inhibiteur de cPLA2α pour une utilisation selon la revendication 1, dans lequel l'inhibiteur de cPLA2α est choisi parmi du N-{(2S,4R)-4-(Biphényl-2-ylméthyl-isobutyl-amino)-1-[2-(2,4-difluorobenzoyl)-benzoyl]-pyrrolidin-2-ylméthyl}-3- [4-(2,4-dioxothiazolidin-5-ylidèneméthyl)-phényl]acrylamide (pyrrolidine-2); l'arachidonyl trifluorométhyl cétone (ATK) ; le méthyl arachidonyl fluorophosphonate (MAFP) ; de la 4-[4-[2-[2-[bis(4-chlorophényl) méthoxy]éthyl-sulfonyl]éthoxy]phényl]-1,1-,1-trifluoro-2-butanone; l'acide 3,3-diméthyl-6-(3-lauroyluréido)-7-oxo-4-thia-1-azabicyclo[3,2,0]heptane-2-carboxylique ; du (N [[2S, 4R)-4-[([1,1'-biphényl]-2-ylméthyl)(2-méthylpropyl)amino]-1-[2-(2,4-difluorobenzoyl)benzoyl]-2-pyrrolidinyl]méthyl]-3-[4-[(2,4-dioxo-5-thiazolidinylidène)méthyl]phényl]-2-propénamide (RSC-3388); l'acide 1-(3-(4-octylphénoxy)-2-oxopropyl)-3-(2,2,2-trifluoroacétyl)-1H-indole-5-carboxylique; l'acide 3-(3-méthyl-1,2,4-oxadiazol-5-yl)-1-(3-(4-octylphénoxy)-2-oxopropyl)-1H-indole-5-carboxylique; l'acide 3-(3-méthyl-1,2,4-oxadiazol-5-yl)-1-(2-oxo-3-(4-phénoxyphénoxy)propyl)-1H-indole-5-carboxylique; l'acide 4-(2-oxohexadécanamido) butanoïque (AX006), l'acide 4-(2-oxododécanamido) octanoïque (AX007); l'éthylester d'acide 4-(2-oxohexadécanamido) butanoïque (AX048), l'acide 4,4-[(2-oxohexadécanamido)-2-méthylpropyl]butanoïque (AX059), et le méthylester d'acide 2-(2-oxohexadécanamido) acétique (AX115).

3. Inhibiteur de cPLA2α pour une utilisation selon la revendication 1 ou 2, dans lequel l'inhibiteur de cPLA2α est du N-{(2S,4R)-4-(Biphényl-2-ylméthyl-isobutyl-amino)-1-[2-(2,4-difluorobenzoyl)-benzoyl]-pyrrolidin-2-ylméthyl}-3-[4-(2,4-dioxothiazolidin-5-ylidèneméthyl)-phényl]acrylamide (pyrrolidine-2).

4. Composition pharmaceutique pour une utilisation dans le traitement ou la prévention d'une infection par un flavivirus du genre Flavi ou du genre Hepaci, laquelle composition pharmaceutique comprend un ou plusieurs inhibiteur(s) de cPLA2α, et, facultativement, au moins un support pharmaceutiquement acceptable et/ou au moins un excipient habituel.

5. Préparation de combinaison pour une utilisation dans le traitement ou la prophylaxie d'une infection par un flavivirus du genre Flavi ou du genre Hepaci contenant au moins un inhibiteur de cPLA2α et au moins un ingrédient pharmaceutique actif supplémentaire.

6. Préparation de combinaison pour une utilisation selon la revendication 5, dans laquelle l'ingrédient pharmaceutique actif supplémentaire est choisi parmi l'interféron pegylé alpha-2a, l'interféron pegylé alpha-2b, la ribavirine, la viramidine, le télaprévir/VX 950, SP 30, ITX 5061, RG7128, PSI-7977, NM 283, Albuferon et/ou Zadaxin.

7. Utilisation d'un inhibiteur de l'alpha phospholipase A2 cytosolique pour la préparation d'un médicament pour prévenir ou traiter une infection par un flavivirus du genre Flavi ou du genre Hepaci.
